# EUROPÄISCHE PATENTANMELDUNG

(11) **EP 4 478 024 A1**
(43) Veröffentlichungstag der Anmeldung: **18.12.2024**
(21) Anmeldenummer: 24181769.1
(22) Anmeldetag: 12.06.2024
(51) Int. Cl.: G01N 1/40, G01N 15/06, G01N 33/00, G01N 5/04, G01N 1/22, G01N 15/00

(54) **VERFAHREN, MESSVORRICHTUNG UND VERWENDUNG EINER HEIZPLATTE, EINES PARTIKELZÄHLERS UND EINES AEROSOLQUALITÄTSSENSORS ZUR QUALITÄTSBESTIMMUNG VON AEROSOLPARTIKELN IN EINEM GASGEMISCH**

(30) Priorität: 13.06.2023 DE 102023115387
(71) Anmelder: Testo SE & Co. KGaA, 79822 Titisee-Neustadt (DE)
(72) Erfinder: Göpfert, Dirk, 79241 Ihringen (DE); Egin, Seyit, 78199 Bräunlingen (DE); Schütze, Andreas, 66386 St. Ingbert (DE)
(74) Vertreter: Mertzlufft-Paufler, Cornelius

(57) **Zusammenfassung**

Die Erfindung schlägt allgemein ein Verfahren sowie eine Messvorrichtung (10) zur Luftqualitätsbestimmung mit Hilfe von Aerosolpartikeln (1) in einem Gasgemisch (2) vor, wobei Aerosolpartikel (1) aufkonzentriert und durch ein Heizelement (7) ausgeheizt werden, und wobei ein Partikelzähler (8) eine Anzahl der Aerosolpartikel (1) bestimmt und wenigstens ein Luftqualitätsbestimmungs-Sensor (9) zumindest organische Bestandteile der Aerosolpartikel (1) und/oder Signalmuster des Gasgemischs (2) vor und nach der Ausheizung ermittelt (Fig. 3) .

## Beschreibung

Die Erfindung betrifft ein Verfahren zur Luftqualitätsbestimmung mit Hilfe von Aerosolpartikeln in einem Gasgemisch.

Bekanntermaßen werden zur vollständigen Analyse von Gasgemischen (z.B. von Raumluft) verschiedene Parameter erfasst. So werden zur vollständigen Bewertung einer Raumluftqualität neben dem Anteil an CO₂ auch die Konzentration der gasförmigen, organischen Verbindungen sowie eine Partikelanzahl ermittelt. Gasförmige, organische Verbindungen können leicht-flüchtig (mittlerer bis niedriger Dampfdruck) oder semi-flüchtig (sehr niedriger Dampfdruck) sein. Semi-flüchtige organische, gasförmige Verbindungen sind oft an Partikeln gebunden und können über diese eingeatmet werden. Bei einer vollständigen Bewertung einer Raumluftqualität werden die semi-flüchtigen organischen, gasförmigen Verbindungen zumeist nicht erfasst. Herkömmlicherweise erfolgt die Erfassung und Analyse gasförmiger, organischer Verbindungen in einem mehrstufigen und zeitintensiven Prozess.

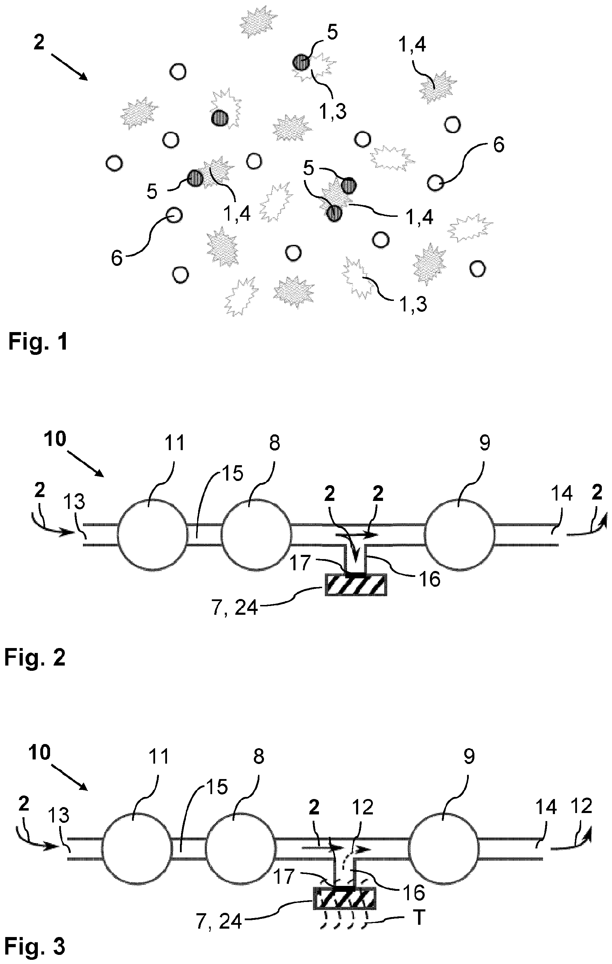

Partikel können anorganischer (kohlenstofffrei; z.B. Salze, Staub) oder organischer (kohlenstoffbasierend; z.B. Ruß, Reifenabrieb) Natur sein. Als Schwebepartikel in einem Gasgemisch werden Partikel häufig als Aerosolpartikel bezeichnet. Eine schematische Darstellung eines Gasgemisches ist in Fig. 1 dargestellt und wird im weiteren Verlauf der Beschreibung, nachfolgend der Figurenaufzählung, beschrieben. Die Einatmung von Aerosolpartikeln kann gesundheitsfördernd (Salze) oder auch gesundheitsschädlich (Ruß, Reifenabrieb) sein. Die Erfassung von Aerosolpartikeln erfolgt zumeist durch Messung einer Anzahl und/oder Masse der Partikel pro Volumen einer bestimmten Größenfraktion eines zu untersuchenden Gasgemisches. Eine Unterscheidung zwischen anorganischen und organischen Aerosolpartikeln wird bei Raumluftqualitätsmessungen üblicherweise nicht vorgenommen.

Zur Bewertung einer Raumluftqualität können beispielsweise organische, gasförmige Verbindungen und Aerosolpartikel separat und in unterschiedlichen Messvorrichtungen bestimmt werden.

Die Erfindung betrifft weiterhin eine Messvorrichtung zur Luftqualitätsbestimmung mit Hilfe von Aerosolpartikeln in einem Gasgemisch.

Die Erfindung betrifft weiter eine Verwendung einer Heizplatte, eines Partikelzählers und eines Luftqualitätsbestimmungs-Sensors, insbesondere eines VOC-Sensors, zur Luftqualitätsbestimmung mit Hilfe von Aerosolpartikeln in einem Gasgemisch.

Es ist daher Aufgabe der Erfindung, die Analyse von Gasgemischen zu verbessern.

Zur Lösung der genannten Aufgabe sind erfindungsgemäß die Merkmale des Anspruchs 1 vorgesehen. Insbesondere wird somit zur Lösung der genannten Aufgabe bei einem Verfahren der eingangs beschriebenen Art erfindungsgemäß vorgeschlagen, dass Aerosolpartikel aufkonzentriert und durch ein Heizelement ausgeheizt werden, und wobei ein Partikelzähler eine Anzahl der Aerosolpartikel bestimmt und wenigstens ein Luftqualitätsbestimmungs-Sensor zumindest organische Bestandteile der Aerosolpartikel vor und nach der Ausheizung ermittelt. Hierdurch können gleichzeitig flüchtige und semi-flüchtige organische, gasförmige Verbindungen und die Zusammensetzung von Aerosolpartikeln in einem Gasgemisch erfasst werden. Somit kann eine Qualitätsbestimmung eines Gasgemisches vorgenommen werden, was die Analyse eines Gasgemisches allgemein verbessert.

Mit Ausheizung wird hier ein kontrolliertes Erhitzen der Aerosolpartikel eines Gasgemisches durch ein Heizelement über einen definierten Zeitraum verstanden. Somit können vorteilhaft die aufkonzentrierten Aerosolpartikel und/oder die daran anhaftenden semi-flüchtigen gasförmigen, organischen Verbindungen verdampfen oder (teilweise) oxidieren, woraufhin deren Verbrennungsprodukte durch wenigstens einen Luftqualitätsbestimmungs-Sensor analysiert werden. Als Luftqualitätsbestimmungs-Sensor ist jeder Sensor zu verstehen, der zumindest organische Verbindungen detektieren kann (z.B. PID-Sensor, Pellistor, GSS-Sensor, Halbleiter-Gassensor).

Bei einer besonders bevorzugten Ausführungsform ist ein VOC-Sensor (Volatile Organic Compounds) als Luftqualitätsbestimmungs-Sensor. Ein VOC-Sensor eignet sich besonders gut zur Luftqualitätsbestimmung durch Ermittlung organische Bestandteile der Aerosolpartikel.

Es kann vorteilhaft vorgesehen sein, dass der Luftqualitätsbestimmungs-Sensor auch anorganische Bestandteile der Aerosolpartikel bzw. anorganische Gase vor und/oder nach der Ausheizung ermittelt. So können beispielsweise durch breitbandige Halbleiter-Gassensoren anorganische Gase wie beispielsweise Wasserstoff und/oder Kohlenstoffmonoxid und/oder Ozon und/oder Stickoxide ermittelt werden. Somit kann die Komplexität der Zusammensetzung eines Gasgemischs noch besser ermittelt werden, sodass eine Luftqualitätsbestimmung besonders verbessert sein kann.

Alternativ oder zusätzlich kann erfindungsgemäß vorgesehen sein, dass der wenigstens eine Luftqualitätsbestimmungs-Sensor ein Signalmuster des Gasgemisches vor und/oder nach der Ausheizung ermittelt. So können bestimmte Signalmuster, beispielsweise Konzentrationsveränderungen von im Gasgemisch befindlicher Komponenten, erfasst werden. So können bestimmte Signalmuster dazu dienen, um ein bestimmtes Gasgemisch, beispielsweise eine Schadstoffquelle wie Dieselabgas oder Holzverbrennungsgase, zu indizieren. Aus dem Signalmuster wird dann insbesondere eine Luftqualitätsbestimmung durchgeführt. Somit können vorteilhaft Luftbestimmungen an Orten, welche keine optimalen und stabilen Luftqualitätsbestimmungen erlauben, durchgeführt werden.

Insbesondere von Vorteil ist die Ermittlung, wenn wenigstens zwei wie hierin beschriebene Luftqualitätsbestimmungs-Sensoren, vorzugsweise ein Sensor-array, zur Luftqualitätsbestimmung verwendet werden. Hierdurch kann die Sensitivität und/oder Selektivität auf bestimmte organische und/oder anorganische Verbindungen sowohl bei der Erfassung insbesondere organischer, gasförmiger Verbindungen (insbesondere semi-flüchtige organische, gasförmige Verbindungen) als auch bei der Erfassung der Zusammensetzung der Aerosolpartikel und/oder der Gaskomponenten des Gasgemisches erhöht werden.

Eine aus dem Signalmuster erfolgende Luftqualitätsbestimmung ist noch sensitiver bzw. selektiver und somit verbessert, wenn die Luftqualitätsbestimmung, wie vorgesehen sein kann, unter Verwendung von Maschinellem Lernen und/oder Künstlicher Intelligenz erfolgt. Hierdurch können die ermittelten Signalmuster abgeglichen und schnell erkannt und zur Luftqualitätsbestimmung zeitnah verwendet werden.

Alternativ können auch mehrere (unterschiedliche) Luftqualitätsbestimmungs-Sensoren (Sensor-Array) zur Qualitätsbestimmung verwendet werden, wodurch die Sensitivität und/oder Selektivität auf bestimmte organische Verbindungen sowohl bei der Erfassung organischer, gasförmiger Verbindungen (insbesondere semi-flüchtige organische, gasförmige Verbindungen) als auch bei der Erfassung der Zusammensetzung der Aerosolpartikel erhöht werden kann.

Bei einem erfindungsgemäßen Verfahren können die Aerosolpartikel so aufkonzentriert werden, dass die Sensitivität der Qualitätsbestimmung des Gasgemisches erhöht wird, wodurch auch wenig abundante organische Verbindungen detektiert werden können.

Ferner von Vorteil ist, dass durch einen Vergleich der Messsignale des wenigstens einen Luftqualitätsbestimmungs-Sensor vor (Bestimmung eines Basissignals) und nach Ausheizung der Anteil der anorganischen und organischen Bestandteile der Aerosolpartikeln bestimmt werden kann.

Weiterhin kann ein erfindungsgemäßes Verfahren vor Ort durchgeführt werden, was zeit- und kostensparend ist, da die Messungen nicht in einem ortsfernen Labor stattfinden müssen.

Bei einer vorteilhaften Ausgestaltung kann vorgesehen sein, dass die Aerosolpartikel vorzugsweise vor der Ausheizung an dem Heizelement aufkonzentriert werden. Somit kann die Effizienz der Ausheizung der Aerosolpartikel gesteigert werden, wodurch vorteilhaft mehr Verbrennungsprodukte pro Zeitintervall entstehen können. Hierdurch kann die Sensitivität zur Erfassung bestimmter organischer Verbindungen noch weiter erhöht werden.

Bei einer vorteilhaften Ausgestaltung kann vorgesehen sein, dass die Ausheizung sprunghaft oder graduell erfolgt. Aufgrund unterschiedlicher Siedepunkte verschiedener organischer Verbindungen kann somit eine Trennung unterschiedlicher Verbindungen erfolgen, welche vorteilhaft die Sensitivität und/oder Selektivität bei der Luftqualitätsbestimmung mit Hilfe der Aerosolpartikel erhöhen kann.

Finden bei einem Verfahren mehrere (verschiedene) Luftqualitätsbestimmungs-Sensoren Verwendung, kann die Qualitätsbestimmung eines Gasgemisches durch eine noch gesteigerte Sensitivität und/oder Selektivität weiter verbessert werden.

Bei einer vorteilhaften Ausgestaltung kann vorgesehen sein, dass zur Bestimmung organischer Bestandteile vor der Ausheizung das Gasgemisch durch einen Nebenzulauf zu dem wenigstens einen Luftqualitätsbestimmungs-Sensor zugeführt wird und dass zur Bestimmung organischer Bestandteile nach der Ausheizung das Gasgemisch durch einen Zulauf zu dem wenigstens einen Luftqualitätsbestimmungs-Sensor zugeführt wird. Hierbei kann die Zufuhr des Gasgemisches hinter dem Heizelement erfolgen. Somit können unerwünschte Akkumulationen von Aerosolpartikeln an der Heizplatte während der Messung des Basissignals vermindert oder vermieden werden. Dadurch werden die Messungen nach der Ausheizung noch genauer, wodurch die Qualitätsbestimmung eines Gasgemisches erhöht werden kann.

Durch beispielsweise Ventile kann eine unerwünschte Zufuhr des Gasgemisches bei der Erfassung des Basissignals (geschlossenes Ventil an dem Zulauf) oder bei der Erfassung organischer Verbindung nach Ausheizung (geschlossenes Ventil an dem Nebenzulauf) verhindert werden.

Alternativ oder zusätzlich kann zur Bestimmung des Basissignals das einströmende Gasgemisch durch eine Filterkonstruktion, insbesondere durch einen Aktivkohlefilter, einem katalytischen Heizelement oder einer Kombination der soeben genannten Elemente, gereinigt werden. Hierdurch wird ein noch genaueres Basissignal bestimmt, wodurch die Qualitätsbestimmung eines Gasgemisches noch weiter verbessert werden kann.

Bei einer vorteilhaften Ausgestaltung kann vorgesehen sein, dass während und/oder nach der Ausheizung ein Masseverlust der Aerosolpartikel ermittelt wird. Der Masseverlust der Aerosolpartikel kann somit vorteilhaft als zusätzlicher Parameter zur Qualitätsbestimmung eines Gasgemisches herangezogen werden.

Ferner kann vorteilhaft sein, dass die Erfassung einer Masse bzw. eines Masseverlusts Aufschluss über die Reinheit der verwendeten Messvorrichtung geben kann, was insbesondere für die Genauigkeit für nachfolgende Messungen wichtig ist.

Insbesondere wird ein Masseverlust der Aerosolpartikel an dem Heizelement ermittelt. Hierbei kann das Heizelement direkt als Mikrowaage ausgebildet sein. Vorteilhaft ist dies insbesondere dann, wenn die Aerosolpartikel an dem Heizelement aufkonzentriert werden.

Bei einer vorteilhaften Ausgestaltung kann vorgesehen sein, dass eine Teilmenge oder die Gesamtmenge eines Gasgemisches dem wenigstens einen Luftqualitätsbestimmungs-Sensor zugeführt wird. Somit kann dem Luftqualitätsbestimmungs-Sensor eine definierte Menge eines Gasgemisches zugeführt werden. Beispielsweise kann dem Luftqualitätsbestimmungs-Sensor zur Bestimmung des Basissignals eine Hälfte der Gesamtmenge und für die Bestimmung eines Signals nach Ausheizung die zweite Hälfte der Gesamtmenge zugeführt werden. Somit kann zur exakten Bestimmung der Qualität eines Gasgemisches die optimale Menge eines Gasgemisches dem oder einem Luftqualitätsbestimmungs-Sensor zugeführt werden.

Zur Lösung der genannten Aufgabe sind erfindungsgemäß die Merkmale des nebengeordneten, auf eine Messvorrichtung gerichteten Anspruchs vorgesehen. Insbesondere wird somit zur Lösung der genannten Aufgabe bei einer Messvorrichtung der eingangs beschriebenen Art erfindungsgemäß vorgeschlagen, dass die Messvorrichtung ein Heizelement, einen Partikelzähler und wenigstens einen Luftqualitätsbestimmungs-Sensor aufweist, wobei das Heizelement dem wenigstens einen Luftqualitätsbestimmungs-Sensor vorgelagert ist. Somit können vorteilhaft gleichzeitig der Anteil gasförmiger, organischer Verbindungen, eine Aerosolpartikelanzahl in einem Gasgemisch sowie ein Anteil von Aerosolpartikeln organischen Ursprungs erfasst werden. Eine erfindungsgemäße Messvorrichtung kann insbesondere dazu ausgebildet sein, ein wie zuvor beschriebenes oder nachfolgend beanspruchtes Verfahren durchzuführen.

Bei einer erfindungsgemäßen Messvorrichtung ist ferner von Vorteil, dass organische, gasförmige Verbindungen und Aerosolpartikel in einer Messvorrichtung gemessen und analysiert werden können, wodurch die Qualitätsbestimmung eines Gasgemisches schneller und kostengünstiger ermittelt werden kann.

Weiterhin kann eine Messvorrichtung derart ausgestaltet sein, dass eine Messung vor Ort und nicht in einem Labor stattfinden kann. Dies ist insbesondere dann von Vorteil, wenn die Raumluftqualität einer Werkstätte, einer Küche oder eines anderen Ortes, an dem organische Aerosolpartikel entstehen, ermittelt werden sollen. Somit kann schneller auf eine zu hohe Aerosolpartikel- bzw. Schadstoffbelastung reagiert werden. Eine erfindungsgemäße Messvorrichtung kann kostengünstig ausgestaltet sein, da sie lediglich ein Heizelement, einen Partikelzähler und wenigstens einen Luftqualitätsbestimmungs-Sensor benötigt. Eine kostengünstige Messvorrichtung kann somit an Privatkunden oder Kleinunternehmer (Autowerkstattbetreiber, Restaurantbesitzer) vertrieben werden.

Das Heizelement ist dem Luftqualitätsbestimmungs-Sensor vorgelagert, da die Verbrennungsprodukte somit einfach mit dem Gasstrom von dem Heizelement zu dem Luftqualitätsbestimmungs-Sensor hingeführt werden können. Somit werden dem Luftqualitätsbestimmungs-Sensor nicht schon vor der Ausheizung unkontrolliert Aerosolpartikel zugeführt, was eine Qualitätsbestimmung eines Gasgemisches verfälschen könnte.

Bei einer vorteilhaften Ausgestaltung kann vorgesehen sein, dass der Partikelzähler dem Heizelement und/oder dem wenigstens einen Luftqualitätsbestimmungs-Sensor nachgelagert ist. Hierdurch können Verschleppungen organischer Verbindungen zu dem Partikelzähler minimiert oder gar verhindert werden, was für die Langlebigkeit des Partikelzählers bzw. der Messvorrichtung von Vorteil ist.

Bei einer vorteilhaften Ausgestaltung kann vorgesehen sein, dass das Heizelement mit einem Absorbermaterial ausgestattet ist. Das Absorbermaterial kann zu einer effizienteren Aufkonzentrierung der Aerosolpartikel führen, wodurch mehr organisches Material verbrannt werden kann, was die Qualitätsbestimmung eines Gasgemisches noch weiter verbessern kann.

Sollte in einer weiteren Ausführungsform die Aufkonzentrierung zusätzlich oder alternativ an einer anderen Stelle der Messvorrichtung stattfinden, kann auch diese Stelle ein Absorbermaterial aufweisen.

Bei einer vorteilhaften Ausgestaltung kann vorgesehen sein, dass das Heizelement als Mikrowaage ausgebildet ist. Somit kann vorteilhaft der Masseverlust der Aerosolpartikel bei der Bestimmung der organischen Bestandteile der Aerosolpartikel registriert werden.

Insbesondere kann das Heizelement als Quarzkristall-Mikrowaage ausgebildet sein. Quarzkristall-Mikrowaagen können besonders stabil bei hohen Temperaturen sein, wodurch die Messvorrichtung langlebiger ausgebildet sein kann. Ferner können Quarzkristall-Mikrowaagen nebst Massebestimmung als Sensoren für weitere Parameter verwendet werden.

Bei einer vorteilhaften Ausgestaltung kann vorgesehen sein, dass die Messvorrichtung wenigstens zwei Luftqualitätsbestimmungs-Sensoren aufweist. Durch die Verwendung mehrerer Luftqualitätsbestimmungs-Sensoren kann die Sensitivität (bei Verwendung von Luftqualitätsbestimmungs-Sensoren gleichen Bautyps) und/oder die Selektivität (bei Verwendung von Luftqualitätsbestimmungs-Sensoren unterschiedlichen Bautyps) von zu messenden organischen Verbindungen erhöht werden.

Alternativ oder zusätzlich kann dabei vorgesehen sein, dass der oder ein", insbesondere der zweite der wenigstens zwei Luftqualitätsbestimmungs-Sensoren, insbesondere VOC-Sensor und/oder ein Photoionisationsdetektor und/oder ein Pellistor und/oder ein Halbleiter-Gassensor ist. Derartige Luftqualitätsbestimmungs-Sensoren sind kommerziell und kostengünstig für die Einbringung in eine Messvorrichtung verfügbar. Somit können Herstellungs- und Reparaturkosten einer Messvorrichtung gering gehalten werden. Ferner sind derartige Luftqualitätsbestimmungs-Sensoren thermostabil ausgebildet, um risikofrei zur Analyse von Gasgemischen unter Erhitzung verwendet werden zu können.

Bei einer vorteilhaften Ausgestaltung kann vorgesehen sein, dass die Messvorrichtung wenigstens eine Pumpe zur Zuführung des Gasgemisches zum Partikelzähler hin aufweist. Somit kann autark durch die Pumpe das Gasgemisch dem Partikelzähler zugeführt werden.

Eine Pumpe kann zum Aufbau und/oder zur Aufrechterhaltung eines definierten Gasstroms verwendet werden. Beispielsweise kann die Pumpe ein definiertes Volumen eines Gasgemisches der Messvorrichtung zuführen.

Insbesondere kann dabei vorgesehen sein, dass die Pumpe dem Luftqualitätsbestimmungs-Sensor nachgelagert ist. Somit kann ein Einfluss der Pumpe auf den VOC-Sensor verringert oder gar vermieden werden. Beispielsweise kann somit ein Eintrag von Schmierstoffen und/ oder Abrieb von Dichtungsmaterial der Pumpe in den Luftqualitätsbestimmungs-Sensor verringert oder gar vermieden werden, sodass Messungen des Luftqualitätsbestimmungs-Sensors besonders genau sein können.

Bei einer vorteilhaften Ausgestaltung kann vorgesehen sein, dass die Messvorrichtung einen Zulauf und einen Nebenzulauf für das oder ein Gasgemisch aufweist. Somit kann zur Bestimmung des Basissignals das Gasgemisch der Messvorrichtung über den Nebenzulauf zur Verfügung gestellt werden. Dabei kann die Hauptleitung (mitsamt Zulauf) insbesondere durch ein Ventil versperrt sein, wodurch die Messgenauigkeit der Messvorrichtung zur Qualitätsbestimmung eines Gasgemisches erhöht wird.

Dabei weist der Nebenzulauf vorzugsweise einen Filter auf, wobei der Filter besonders bevorzugt als ein katalytisches Heizelement und/oder als ein Aktivkohlefilter ausgebildet ist. Durch derartige oder weitere dem Fachmann bekannte Filter kann ein Gasgemisch gereinigt werden, wodurch eine noch präzisere Bestimmung des Basissignals und somit eine noch verbesserte Qualitätsbestimmung erreicht werden kann.

Bei einer vorteilhaften Ausgestaltung kann vorgesehen sein, dass die Messvorrichtung wenigstens zwei Hauptleitungen aufweist. Dabei weist jede Hauptleitung wenigstens einen Partikelzähler und einen Luftqualitätsbestimmungs-Sensor auf. Somit können die Erfassung eins Basissignals und die Erfassung eines Signals nach Ausheizung parallel erfolgen, wodurch Messungen noch genauer und schneller ausgeführt werden können.

Zur Kosteneinsparung verfügt vorzugsweise lediglich eine Hauptleitung zur Erfassung eines Signals nach Ausheizung über ein Heizelement.

Zur Lösung der genannten Aufgabe sind erfindungsgemäß die Merkmale des nebengeordneten, auf eine Verwendung gerichteten Anspruchs vorgesehen. Insbesondere wird somit zur Lösung der genannten Aufgabe bei einer Verwendung der eingangs beschriebenen Art erfindungsgemäß vorgeschlagen, dass vorzugsweise gleichzeitig eine Aerosolpartikelanzahl und ein Anteil organischer Bestandteile von Aerosolpartikeln eines Gasgemisches bestimmt werden. Hierdurch kann schnell und kostengünstig die Qualität eines Gasgemisches ermittelt werden, was von Vorteil ist.

Die Erfindung wird nun anhand von Ausführungsbeispielen näher beschrieben, ist jedoch nicht auf die Ausführungsbeispiele beschränkt. Weitere Ausführungsbeispiele ergeben sich durch Kombination der Merkmale einzelner oder mehrerer Ansprüche untereinander und/oder mit einzelnen oder mehreren Merkmalen der Ausführungsbeispiele.

Es zeigen:
- Fig. 1: ein Ausschnitt eines Gasgemisches,
- Fig. 2: eine erfindungsgemäße Messvorrichtung während der Erfassung des Basissignals,
- Fig. 3: eine erfindungsgemäße Messvorrichtung während der Ausheizung,
- Fig. 4: eine alternative Ausführungsform einer erfindungsgemäßen Messvorrichtung,
- Fig. 5: eine weitere alternative Ausführungsform einer erfindungsgemäßen Messvorrichtung,
- Fig. 6: eine alternative Ausführungsform einer erfindungsgemäßen Messvorrichtung mit einem Nebenzulauf und einem katalytischen Heizelement,
- Fig. 7: eine alternative Ausführungsform einer erfindungsgemäßen Messvorrichtung mit einem Nebenzulauf und einem Aktivkohlefilter,
- Fig. 8: eine alternative Ausführungsform einer erfindungsgemäßen Messvorrichtung mit zwei Hauptleitungen.

Ein Ausschnitt eines zu untersuchenden Gasgemisches 2 ist in Fig. 1 dargestellt. Wie eingangs schon erläutert, können sich in einem Gasgemisch 2 verschiedene Aerosolpartikel 1, 3, 4 befinden. Aerosolpartikel 1 können anorganisch 3 oder organisch 4 sein, wobei vor allem organische Aerosolpartikel 1, 4 (wie Ruß oder Reifenabrieb) gesundheitsschädlich sind. Semi-flüchtige organische Verbindungen 5 können an Aerosolpartikeln 1, 3, 4 anhaften und über diese eingeatmet werden und ebenfalls die Gesundheit beeinträchtigen. Leicht-flüchtige organische Verbindungen 6 schweben zumeist losgelöst von Aerosolpartikeln 1, 3, 4 in dem Gasgemisch 2. Um eine umfassende Qualitätsbestimmung eines Gasgemisches 2, insbesondere einer Raumluft, zu ermitteln, sollten viele, unter anderem die eingangs beschriebenen, Parameter eines Gasgemisches 2 gemessen werden.

Das in den Figuren 2 und 3 dargestellte Verfahren, oder auch die zuvor beschriebenen oder nachfolgend beanspruchten Verfahren, erweitern die Qualitätsbestimmung eines Gasgemisches 2 dahingehend, da hierbei gleichzeitig der Anteil gasförmiger, organischer Verbindungen 5, 6, eine Anzahl an Aerosolpartikeln 1, 3, 4, sowie ein Anteil von Aerosolpartikeln 1, 4 organischen Ursprungs erfasst werden können.

Fig. 2 und Fig. 3 zeigen eine Ausführungsform einer erfindungsgemäßen Messvorrichtung 10 vor (Fig. 2) und während der Ausheizung (Fig. 3) eines Gasgemisches 2. Das in diesen Figuren dargestellte Verfahren kann wie folgt ablaufen.

Ein definiertes Gasgemisch 2, beispielsweise ein definiertes Volumen des Gasgemisches 2, wird mit Hilfe einer Pumpe 11 von einem Zugang 13 durch eine Hauptleitung 15 zu einem Partikelzähler 8 geführt, welcher die Anzahl an Aerosolpartikeln 1, 3, 4 des Gasgemisches 2 registriert.

Die Messvorrichtung 10 weist eine Einbuchtung 16, zu dem Heizelement 7 hinführend, auf. Die Einbuchtung 16 kann so ausgebildet sein, dass ein Gasgemisch 2, insbesondere eine Teilmenge eines Gasgemisches 2, dem Heizelement 7, 24 zugeführt werden kann, sodass Aerosolpartikel 1, 3, 4 aufkonzentriert werden können.

Dem Heizelement 7, 24 wird eine Teilmenge oder die Gesamtmenge des definierten Gasgemisches 2 zugeführt, sodass Aerosolpartikel 1, 3, 4 an dem Heizelement 7, 24 anhaften können. Dabei kann das Heizelement 7, 24, wie in Fig. 2 und Fig. 3 dargestellt, ein Absorbermaterial 17 aufweisen, welches die Anhaftung der Aerosolpartikel 1, 3, 4 noch weiter verstärkt.

Zur Aufkonzentrierung der Aerosolpartikel 1, 3, 4 können auch zusätzliche oder alternative Anhaftungstechniken Verwendung finden. So kann ein Teil der Hauptleitung 15 angeraut bzw. so ausgebildet sein, dass die Aerosolpartikel 1, 3, 4 daran haften. Zusätzlich kann dieser Teil der Hauptleitung 15 teilweise oder vollständig als Heizelement 7, 24 ausgebildet sein.

Im Falle des in den Figuren 2 und 3 beschriebenen Verfahrens wird durch das Heizelement 7, welches als Mikrowaage 24 ausgebildet ist, zusätzlich ein Masseverlust der Aerosolpartikel 1, 3, 4 während der Ausheizung (Fig. 3) registriert. Die hier gezeigte Mikrowaage 24 ist eine Quarzkristallwaage, kann jedoch auch jede andere dem Fachmann bekannte Mikrowaage 24 sein.

Das Gasgemisch 2 wird während bzw. nach Ausheizung 12 (gestrichelter Pfeil) an wenigstens einem Luftqualitätsbestimmungs-Sensor 9 vorbeigeführt, wo organische Komponenten detektiert werden. Als Luftqualitätsbestimmungs-Sensoren 9 können insbesondere ein Photoionisationsdetektor und/oder ein Pellistor und/oder ein vorzugsweise oxidischer Halbleiter-Gasensor eingesetzt werden.

In den Ausführungen gezeigt ist zumindest ein VOC-Sensor als Luftqualitätsbestimmungssensor 9.

Der in Fig. 2 gezeigte Verfahrensschritt erfolgt ohne Ausheizung, weswegen der wenigstens eine Luftqualitätsbestimmungs-Sensor 9 hauptsächlich die leichtflüchtigen organischen Verbindungen 6 und gegebenenfalls anorganische Verbindungen detektiert. Vor der Ausheizung wird durch diesen Verfahrensschritt also ein Basissignal ermittelt, das unter anderem eine Anzahl von Aerosolpartikeln 1, 3, 4 und eine Konzentration leicht-flüchtiger organischer Verbindungen 6 beinhaltet.

Über einen Ablauf 14 wird das Gasgemisch 2 von der Messvorrichtung 10 ab- bzw. weitergeführt.

Nach einem bestimmten Registrierungsintervall (Basissignal) wird das Heizelement 7 erwärmt (Fig. 3). Über einen bestimmten Zeitraum und bei steigender bzw. erhöhter Temperatur T verdampfen organische Verbindungen der Aerosolpartikel 1, 4 bzw. die semi-flüchtigen organischen Verbindungen 5 des Gasgemisches 2. Deren Verbrennungsprodukte werden anschließend von dem wenigstens einen Luftqualitätsbestimmungs-Sensor 9 detektiert.

Je nach Applikationsbereich kann die Messvorrichtung 10 so eingerichtet sein, dass der Anstieg der Temperatur T graduell oder sprunghaft erfolgt. Aufgrund unterschiedlicher physikalischer Eigenschaften verschiedener organischer Komponenten kann somit eine höhere Selektivität bzw. Sensitivität des Verfahrens bzw. einer Messvorrichtung 10 erreicht werden. Somit können und werden auch Signalmuster des Gasgemisches während des Verfahrens ermittelt.

Durch Vergleich der Signale des wenigstens einen Luftqualitätsbestimmungs-Sensors 9 vor (Fig. 2; Basissignal) und nach der Ausheizung (Fig. 3) kann der Anteil organischer, gasförmiger Verbindungen 5 an den Aerosolpartikeln 1, 3, 4 bestimmt werden. Durch die Registrierung des Massenverlusts der Aerosolpartikel 1, 3, 4 durch die heizbare Mikrowaage 7, 24 wird die Bestimmung der organischen Komponenten der Aerosolpartikel 1,3, 4 noch genauer ermittelt.

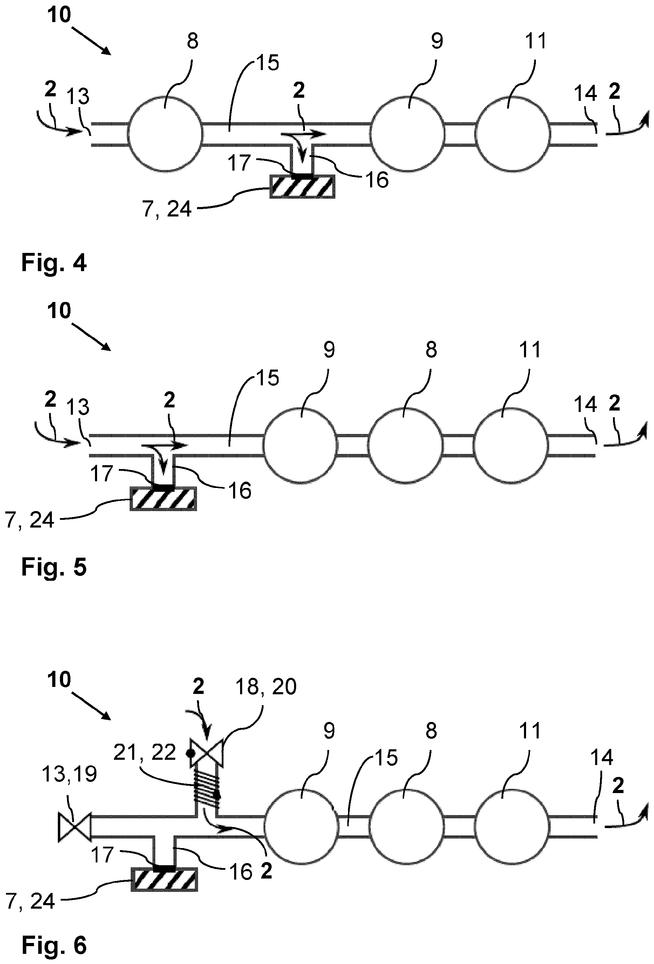

In den Figuren 4 bis 8 sind alternative Messvorrichtungen 10 zu der in den Figuren 2 und 3 dargestellten Messvorrichtung 10 offenbart. Auch diese alternativen Messvorrichtungen 10 können das soeben beschriebene Verfahren bzw. die zuvor beschriebenen oder nachfolgend beanspruchten Verfahren, durchführen. Funktionell und/oder konstruktiv zu dem vorangehenden Ausführungsbeispiel gleichartige oder identische Bauteile und Funktionseinheiten sind mit denselben Bezugszeichen bezeichnet und nicht noch einmal gesondert beschrieben. Die Ausführungen zu den Figuren 2 und 3 gelten daher zu den Figuren 4 bis 8 entsprechend.

Das Ausführungsbeispiel nach Fig. 4 unterscheidet sich von dem vorangehenden Ausführungsbeispiel (der Figuren 2 und 3) dadurch, dass die Pumpe 11 dem Luftqualitätsbestimmungs-Sensor 9 nachgelagert ist.

In den Ausführungsbeispielen nach den Figuren 2/3 sowie Figur 4 ist das Heizelement 7, 24 zwischen Partikelzähler 8 und Luftqualitätsbestimmungs-Sensor 9 positioniert. Durch die dem Luftqualitätsbestimmungs-Sensor 9 nachgelagerte Pumpe 11 ist ein Eintrag von Schmierstoffen und Abrieb von Dichtungsmaterial der Pumpe 11 in den Luftqualitätsbestimmungs-Sensor 9 vermieden, sodass die Messungen des Luftqualitätsbestimmungs-Sensors 9 besonders genau sind.

Ebenso kann eine Verschleppung von Aerosolpartikeln 1, 3, 4 zum Partikelsensor 8 verringert bzw. vermieden werden, wenn der Partikelzähler 8 dem Heizelement 7, 24 nachgelagert ist (Fig. 5).

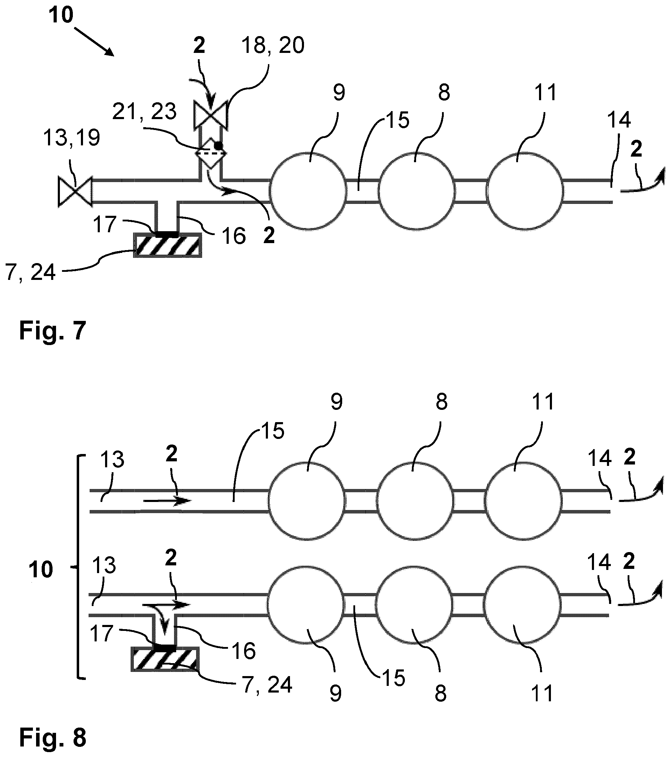

Die in den Figuren 6 und 7 dargestellten Messvorrichtungen 10 bieten alternative Möglichkeiten zur Bestimmung eines Basissignals gegenüber den vorher beschriebenen Messvorrichtungen 10. Bei den alternativen Messvorrichtungen 10 wird das Gasgemisch 2 durch einen Nebenzulauf 18 dem Luftqualitätsbestimmungs-Sensor 9 zugeführt. Der Nebenzulauf 18 ist zwischen Heizelement 7, 24 und Luftqualitätsbestimmungs-Sensor 9 positioniert. Während der Messung des Basissignals ist der Zulauf 13 durch ein Hauptventil 19 verschlossen. Ein Ventil 20 des Nebenzulaufs 18 dient zur Regulierung der Zufuhr des Gasgemisches 2.

Um ein noch genaueres Basissignal zu bestimmen, welches zu einer noch exakteren Qualitätsbestimmung eines Gasgemisches 2 führen kann, kann das Gasgemisch 2 vor der Ausheizung durch einen Filter 21 gereinigt werden. Dieser Filter 21 kann ein katalytisches Heizelement 22 (Fig. 6), ein Aktivkohlefilter 23 (Fig. 7), eine Kombination beider zuvor beschriebenen Filter 21 sein, oder ein anderer dem Fachmann bekannter Filter 21. Ein derartiger Filter 21 kann auch in Messvorrichtungen 10 ohne Nebenzulauf 18 integriert sein, wie sie in den Figuren 2 bis 5 und Fig. 8 dargestellt sind.

Alternativ kann ein Basissignal auch durch eine Messvorrichtung 10 mit wenigstens zwei vorzugsweise voneinander separierten Hauptleitungen 15, welche jeweils wenigstens einen Partikelzähler 8 und einen Luftqualitätsbestimmungs-Sensor 9 aufweisen, bestimmt werden (Fig. 8). Dabei wird eine erste Hauptleitung 15 zur Bestimmung des Basissignals verwendet, während eine zweite Hauptleitung 15 Messsignale organischer Komponenten des Gasgemisches 2 nach erfolgter Ausheizung erfasst. Die hier gezeigte erste Hauptleitung 15 weist kein Heizelement 7, 24 auf, wodurch die Messvorrichtung 10 kostengünstiger ausgestaltet sein kann. Zur Qualitätsbestimmung des Gasgemisches 2 werden die Signale des wenigstens einen in der ersten Hauptleitung 15 positionierten Luftqualitätsbestimmungs-Sensors 9 mit dem des wenigstens einen Luftqualitätsbestimmungs-Sensors 9 der zweiten Hauptleitung 15 miteinander verglichen. Bei der in Fig. 8 gezeigten Ausführungsform können ein Basissignal und ein Signal nach der Ausheizung eines Gasgemisches 2 parallel gemessen werden, wodurch die Qualität eines Gasgemisches 2 noch schneller bestimmt werden kann.

In den beschriebenen Verfahren werden auch anorganische Komponenten des Gasgemisch 2 durch die Messvorrichtung 10 gemessen, wie beispielsweise Stickoxide und/oder Wasserstoff.

Die Erfindung schlägt somit allgemein ein Verfahren sowie eine Messvorrichtung 10 zur Qualitätsbestimmung von Aerosolpartikeln 1 in einem Gasgemisch 2 vor, wobei Aerosolpartikel 1 aufkonzentriert und durch ein Heizelement 7 ausgeheizt werden, und wobei ein Partikelzähler 8 eine Anzahl der Aerosolpartikel 1 bestimmt und wenigstens ein Luftqualitätsbestimmungs-Sensor 9 zumindest organische Bestandteile der Aerosolpartikel 1 und/oder Signalmuster des Gasgemischs 2 vor und nach der Ausheizung ermittelt. Dieses Verfahren sowie die Messvorrichtung sind insbesondere zur Qualitätsbestimmung einer Raumluftqualität geeignet, jedoch nicht auf diese Anwendung beschränkt.

### Bezugszeichenliste

- 1: Aerosolpartikel
- 2: Gasgemisch
- 3: anorganische Aerosolpartikel
- 4: organische Aerosolpartikel
- 5: semi-flüchtige organische Verbindungen
- 6: leicht-flüchtige organische Verbindungen
- 7: Heizelement
- 8: Partikelzähler
- 9: Luftqualitätsbestimmungs-Sensor
- 10: Messvorrichtung
- 11: Pumpe
- 12: Gasgemisch während Ausheizung
- 13: Zulauf
- 14: Ablauf
- 15: Hauptleitung
- 16: Einbuchtung
- 17: Absorbermaterial
- 18: Nebenzulauf
- 19: Hauptventil
- 20: Ventil des Nebenzulaufs 18
- 21: Filter
- 22: katalytisches Heizelement
- 23: Aktivkohlefilter
- 24: Mikrowaage

- T: Temperatur

## Patentansprüche

1. Verfahren zur Luftqualitätsbestimmung mit Hilfe von Aerosolpartikeln (1) in einem Gasgemisch (2), wobei Aerosolpartikel (1) aufkonzentriert und durch ein Heizelement (7) ausgeheizt werden, und wobei ein Partikelzähler (8) eine Anzahl der Aerosolpartikel (1) bestimmt und wenigstens ein Luftqualitätsbestimmungs-Sensor (9) zumindest organische Bestandteile der Aerosolpartikel (1) und/oder Signalmuster des Gasgemischs (2) vor und nach der Ausheizung ermittelt.

2. Verfahren nach Anspruch 1, **dadurch gekennzeichnet, dass** die Aerosolpartikel (1) vorzugsweise vor der Ausheizung an dem Heizelement (7) aufkonzentriert werden.

3. Verfahren nach einem der vorhergehenden Ansprüche, **dadurch gekennzeichnet, dass** die Ausheizung sprunghaft oder graduell erfolgt.

4. Verfahren nach einem der vorhergehenden Ansprüche, **dadurch gekennzeichnet, dass** zur Bestimmung organischer Bestandteile vor der Ausheizung das Gasgemisch (2) durch einen Nebenzulauf (18) zu dem wenigstens einen Luftqualitätsbestimmungs-Sensor (9) zugeführt wird und/oder dass zur Bestimmung organischer Bestandteile nach der Ausheizung das Gasgemisch (2) durch einen Zulauf (13) zu dem wenigstens einen Luftqualitätsbestimmungs-Sensor (9) zugeführt wird.

5. Verfahren nach einem der vorhergehenden Ansprüche, **dadurch gekennzeichnet, dass** während und/oder nach der Ausheizung ein Masseverlust der Aerosolpartikel (1), insbesondere ein Masseverlust der Aerosolpartikel (1) an dem Heizelement (7), ermittelt wird.

6. Verfahren nach einem der vorhergehenden Ansprüche, **dadurch gekennzeichnet, dass** eine Teilmenge oder die Gesamtmenge eines Gasgemisches (2) dem wenigstens einen Luftqualitätsbestimmungs-Sensor (9) zugeführt wird.

7. Messvorrichtung (10) zur Luftqualitätsbestimmung mit Hilfe von Aerosolpartikeln (1) in einem Gasgemisch (2), insbesondere nach einem der zuvor beanspruchten Verfahren, aufweisend ein Heizelement (7), einen Partikelzähler (8) und wenigstens einen Luftqualitätsbestimmungs-Sensor (9), wobei das Heizelement (7) dem wenigstens einen Luftqualitätsbestimmungs-Sensor (9) vorgelagert ist.

8. Messvorrichtung (10) nach Anspruch 7, **dadurch gekennzeichnet, dass** der Partikelzähler (8) dem Heizelement (7) und/oder dem wenigstens einen Luftqualitätsbestimmungs-Sensor (9) nachgelagert ist.

9. Messvorrichtung (10) nach einem auf eine Messvorrichtung (10) gerichteten Ansprüche, **dadurch gekennzeichnet, dass** das Heizelement (7) mit einem Absorbermaterial (17) ausgestattet ist.

10. Messvorrichtung (10) nach einem auf eine Messvorrichtung (10) gerichteten, vorangehenden Anspruch, **dadurch gekennzeichnet, dass** das Heizelement (7) als Mikrowaage (23), insbesondere als Quarzkristall-Mikrowaage, ausgebildet ist.

11. Messvorrichtung (10) nach einem auf eine Messvorrichtung (10) gerichteten, vorangehenden Anspruch, **dadurch gekennzeichnet, dass** die Messvorrichtung (10) wenigstens zwei Luftqualitätsbestimmungs-Sensoren (9) aufweist, und/oder wobei der oder ein Luftqualitätsbestimmungs-Sensor (9) insbesondere VOC-Sensor und/oder ein Photoionisationsdetektor und/oder ein Pellistor und/oder ein Halbleiter-Gassensor ist und/oder umfasst.

12. Messvorrichtung (10) nach einem auf eine Messvorrichtung (10) gerichteten, vorangehenden Anspruch, **dadurch gekennzeichnet, dass** die Messvorrichtung (10) wenigstens eine Pumpe (11) zur Zuführung des Gasgemisches (2) zum Partikelzähler (8) hin aufweist, insbesondere wobei die Pumpe (11) dem Luftqualitätsbestimmungs-Sensor (9) nachgelagert ist.

13. Messvorrichtung (10) nach einem auf eine Messvorrichtung (10) gerichteten, vorangehenden Anspruch, **dadurch gekennzeichnet, dass** die Messvorrichtung (10) einen Nebenzulauf (18) für das oder ein Gasgemisch (2) aufweist, dass der Nebenzulauf (18) vorzugsweise einen Filter (21) aufweist, wobei der Filter (21) besonders bevorzugt als ein katalytisches Heizelement (22) und/oder als ein Aktivkohlefilter (23) ausgebildet ist.

14. Messvorrichtung (10) nach einem auf eine Messvorrichtung (10) gerichteten, vorangehenden Anspruch, **dadurch gekennzeichnet, dass** die Messvorrichtung (10) wenigstens zwei Hauptleitungen (15) aufweist.

15. Verwendung einer Heizplatte (7), eines Partikelzählers (8) und eines Luftqualitätsbestimmungs-Sensors (9), insbesondere eines VOC-Sensors, zur Luftqualitätsbestimmung mit Hilfe von Aerosolpartikeln (1) in einem Gasgemisch (2), wobei vorzugsweise gleichzeitig eine Aerosolpartikelanzahl und ein Anteil organischer Bestandteile von Aerosolpartikeln (1) eines Gasgemisches (2) bestimmt werden.
